# EUROPÄISCHE PATENTSCHRIFT

(11) **EP 1 360 896 B1**
(45) Veröffentlichungstag und Bekanntmachung des Hinweises auf die Patenterteilung: **22.03.2006**
(21) Anmeldenummer: 03010076.2
(22) Anmeldetag: 03.05.2003
(51) Int. Cl.: A01K 13/00, A46B 15/00

(54) **Bürsten für Tiere**
Animal brush
Brosse pour animaux

(30) Priorität: 07.05.2002 US 378539 P
(43) Veröffentlichungstag der Anmeldung: 12.11.2003
(73) Patentinhaber: Rolf C. Hagen, Inc., Montreal, Quebec H4R 1E8 (CA)
(72) Erfinder: Plante, Robin, Sainte-Barbe, Quebec (CA); Dery, Paul, Montreal H2P 2E7, Quebec (CA)
(74) Vertreter: Meyer, Ludgerus

(56) Entgegenhaltungen:
- GB-A- 970 524
- US-A- 2 220 391
- US-A- 5 365 881
- US-B1- 6 196 235

## Beschreibung

Die Erfindung bezieht sich auf eine Tierpflegebürste und insbesondere auf eine Kombination einer Pflegebürste mit einem Kamm, die einfach zu benutzen ist und eine maximale Pflegewirksamkeit entfaltet.

Aus der GB-A-970 524 ist eine Pflegebürste mit einem Handgriff, einem Spalt in dem Handgriff und einem Pflegekamm, der in dem Spalt aufnehmbar ist, bekannt geworden, die einen an dem Handgriff befestigten Bürstenkopf trägt. Diese Bürste ist insbesondere für stark behaarte Tiere nicht verwendbar, weil sie eine im Wesentlichen ebene Bürstenfläche enthält, die den Anforderungen der Tierpflege nicht genügt.

In der US-A-2 220 391 ist eine Bürste gezeigt, die eine konvexe Arbeitsfläche mit Zinken aufweist, die jedoch ebenfalls nicht ergonomisch gestaltet ist und außerdem nicht mit einem Kamm kombiniert ist.

Schließlich zeigt die US-A-5 365 881 eine Bürste mit einem konvex gestalteten Bürstenkopf mit einer Reihe von im Bürstenkopf angeordneten Zinken. Auch hierbei ist keine Kombination mit einem Kamm vorhanden.

Eine Bürste, die einfach zu benutzen ist und eine maximale Pflegewirksamkeit erfüllt, ist im Anspruch 1 angegeben. Sie ist leicht, weist einen bequem geformten Handgriff auf, enthält eine konvexe Arbeitsfläche, die eine größere Wirksamkeit als flache Bürsten aufweist, die normalerweise verwendet werden, enthält kräftige Borsten, die besonders effektiv für stark behaarte Tiere sind, weist eine große Arbeitsfläche mit sehr dünnen Drahtzinken für das Kämmen von Oberflächenhaar und Haar mittlerer Tiefe auf und hält die Haare, die beim Kämmen gelöst werden. Zwischen Handgriff und Bürstenkopf ist ein Winkel ausgebildet, der eine unnötige Belastung der Handmuskeln und Handgelenkbändem vermeidet, die beim häufigen Pflegen von Tieren auftritt. Das Pflegewerkzeug weist auch einen bequemen Aufnahmebereich im Handgriff für einen Kamm auf, der zur Feinpflege verwendet wird.

Die Vorteile der vorliegenden Erfindung werden aus der nachfolgenden Beschreibung deutlich.

Jeder der Aspekte der Erfindung trägt unabhängig zur Wirksamkeit der Anordnung aus Bürste und Kamm bei und die Erfindung ist nicht auf eine Anordnung beschränkt, die alle diese Merkmale aufweist.

Die begleitenden Zeichnungen sind nicht maßstabsgerecht. In den Zeichnungen ist jede identische oder nahezu identische Komponente in den verschiedenen Figuren mit der gleichen Ziffer versehen. Zur Verdeutlichung ist nicht jedes Teil in jeder Zeichnung beziffert. In den Zeichnungen zeigen:
- Fig. 1: eine perspektivische Explosionszeichnung einer Anordnung einer Tierpflegebürste nach der Erfindung mit einem aus dem Handgriff entfemten Kamm,
- Fig. 2: eine Aufsicht auf eine Pflegebürste, bei der der Kamm im Spalt des Handgriffs angeordnet ist,
- Fig. 3: eine vergrößerte perspektivische Ansicht des Handgriffs der Anordnung, die Details des Kammspaltes zeigt,
- Fig. 4: eine Teilquerschnittsansicht des Handgriffs, die den Kamm im Handgriffspalt entlang der Linie 4-4 von Fig. 2 zeigt,
- Fig. 5: eine Querschnittsansicht des Handgriffs und des Kamms entlang der Linie 5-5 von Fig. 2,
- Fig. 6: eine teilperspektivische Ansicht des Kopfes der Anordnung, die von der Unterseite des Bürstenkopfes gezeigt ist und einen Teil der kräftigen Drahtborsten und eine Reihe von feinen Drahtborsten gemäß der Erfindung zeigt, und
- Fig. 7: eine Unteransicht der Tierpflegebürste, wobei der Bereich der feinen Drähte der Bürste in unterbrochenen Linien angedeutet ist.

Die Erfindung ist in der Anwendung nicht auf die Details der Konstruktion und der Anordnung der Komponenten gemäß der folgenden Beschreibung oder der Zeichnungen beschränkt. Die Erfindung ist auch in anderen Ausführungsformen geeignet und auf unterschiedliche Weise verwendbar. Die Begriffe und die Terminologie, die zum Zwecke der Beschreibung verwendet werden, sind nicht als Beschränkung zu betrachten. Die Verwendung von "einschließlich", "enthält", "weist auf", "ergibt" und andere Begriffe schließt die nachfolgenden Merkmale und Äquivalente dazu sowie zusätzliche Merkmale ein.

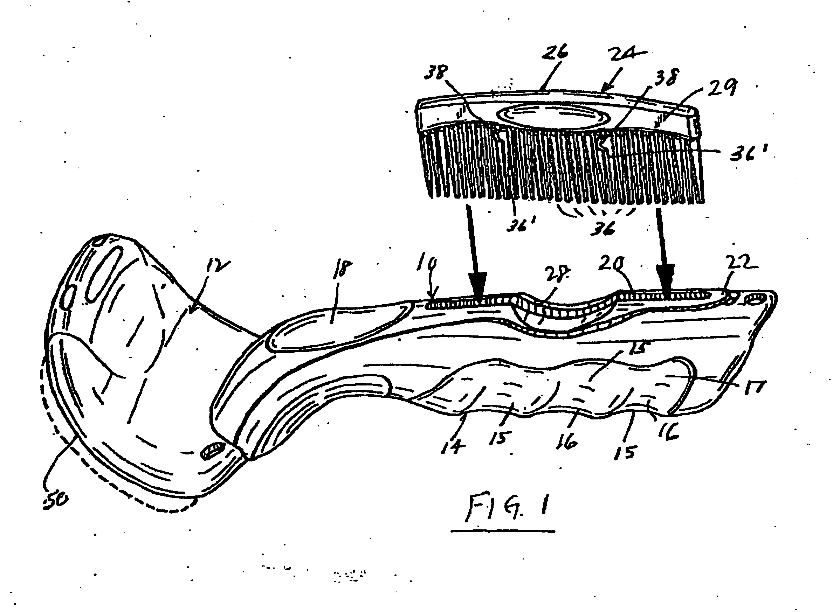

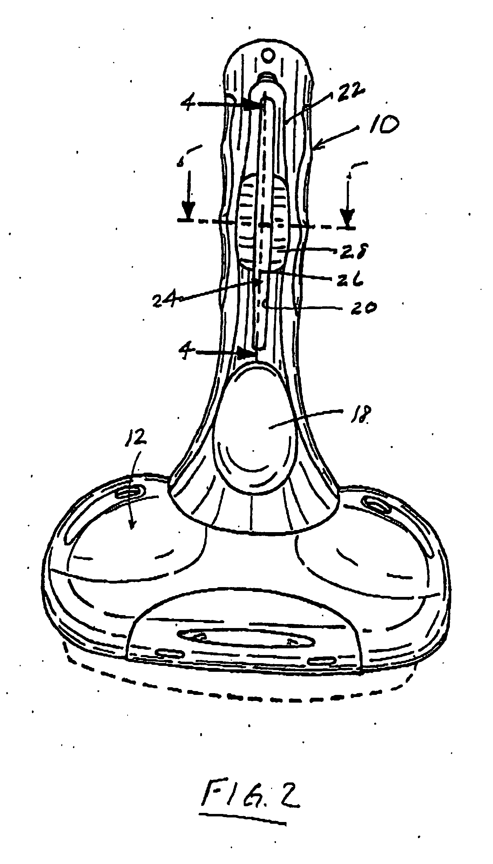

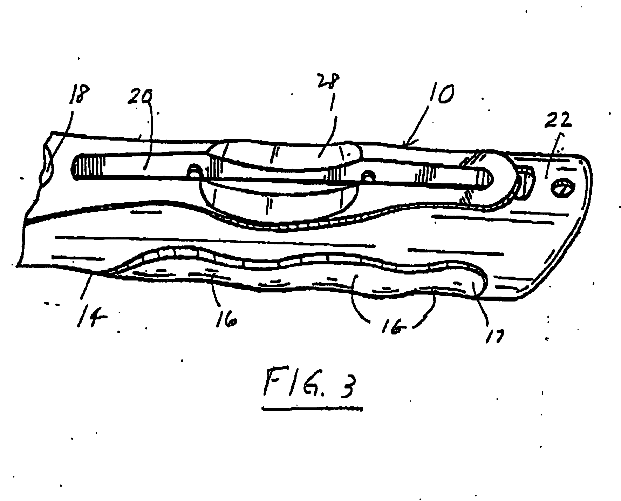

Die Pflegebürstenanordnung nach der vorliegenden Erfindung enthält einen Handgriff 10 und einen Bürstenkopf 12, die separat ausgebildet und miteinander verbunden sein können oder alternativ als einheitliches Gebilde hergestellt sein können. Gemäß einem Aspekt der vorliegenden Erfindung ist der Handgriff 10 vorzugsweise gemäß den Figuren 1 - 3 und 7 ausgeformt, so dass dieser einen besonderen Komfort und eine verbesserte Griffigkeit der Ausbildung ermöglicht. Die untere Fläche 14 des Handgriffs ist, wie bei 16 gezeigt, ausgeformt und die spezielle Fingerkontur 15 erstreckt sich nach oben entlang der Handgriffseiten. Für weiteren Komfort ist der Handgriff vorzugsweise mit elastomerem Kunststoff (s. Figuren 1, 3 und 5 bei 17) auf einem vergleichsweise festen Handgriffgestell aus Polypropylen oder anderem geeigneten Material überzogen. Eine Daumenvertiefung 18 ist vorzugsweise an der oberen Fläche 22 des Handgriffs nahe dem Kopfende angeordnet, um die Griffigkeit des Handgriffs weiter zu verbessern.

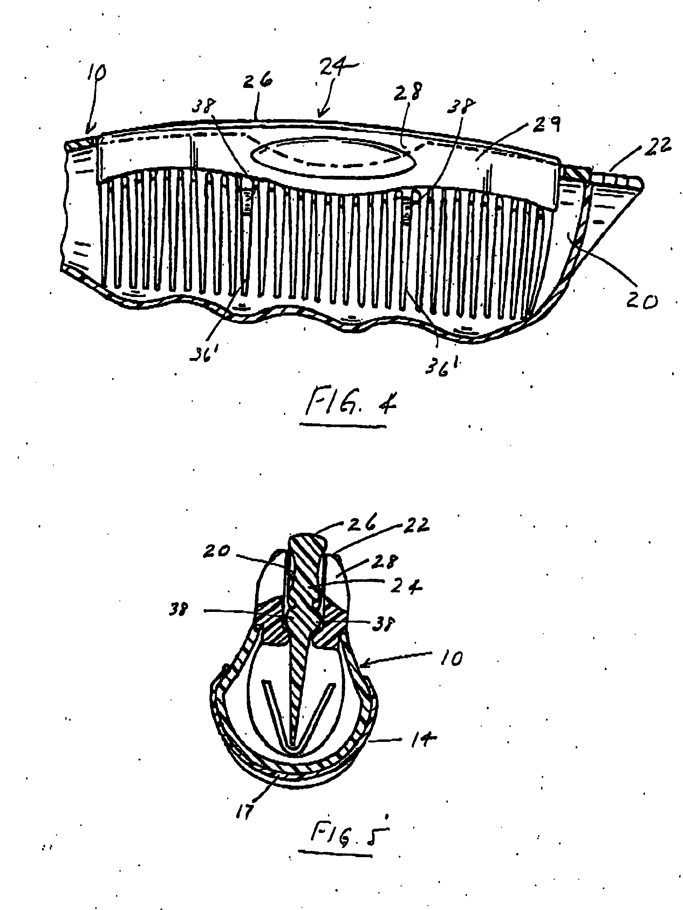

Der Handgriff 10 der dargestellten Ausführungsform enthält einen länglichen Spalt 20, der auf der Oberseite 22 offen ausgebildet ist, und der eine solche Größe besitzt, dass er einen Pflegekamm 24 gemäß den Figuren 1 und 4 aufnehmen kann. Wenn der Kamm in den Spalt 20 eingesetzt ist, erstreckt sich die obere Kante 26 des Kamms mit dem Handgriff 29 nur wenig aus dem Spalt 20, so dass er nicht mit der Griffigkeit des Handgriffs interferiert. Außerdem ist in der oberen Fläche des Bürstenhandgriffs 10 eine flache Ausnehmung 28 ausgebildet und erstreckt sich über die Oberseite des Spalts 20, so dass ein Bereich an den Seiten des Kammhandgriffs 29 ausgebildet ist, um das Einsetzen und Entfernen des Kamms aus und in den Spalt zu erleichtern. Damit der Kamm 24 in dem Spalt 20 gehalten bleibt und nicht zufällig herausfällt und verloren geht, wölben sich einige der Zinken 36, die gemeinsam durch die Ziffer 36' gekennzeichnet sind, nach außen, wie bei 38 gezeigt (s. Figur 5), um eine Klemmung gegen die Seiten des Spaltes zu bewirken und daher in Reibverbindung mit den Spaltwänden stehen, um den Kamm im Spalt festzuhalten. Abhängig von der Form und Größe des Handgriffs kann der Spalt 20 alternativ in den Seiten oder an der Unterseite des Handgriffs angeordnet sein.

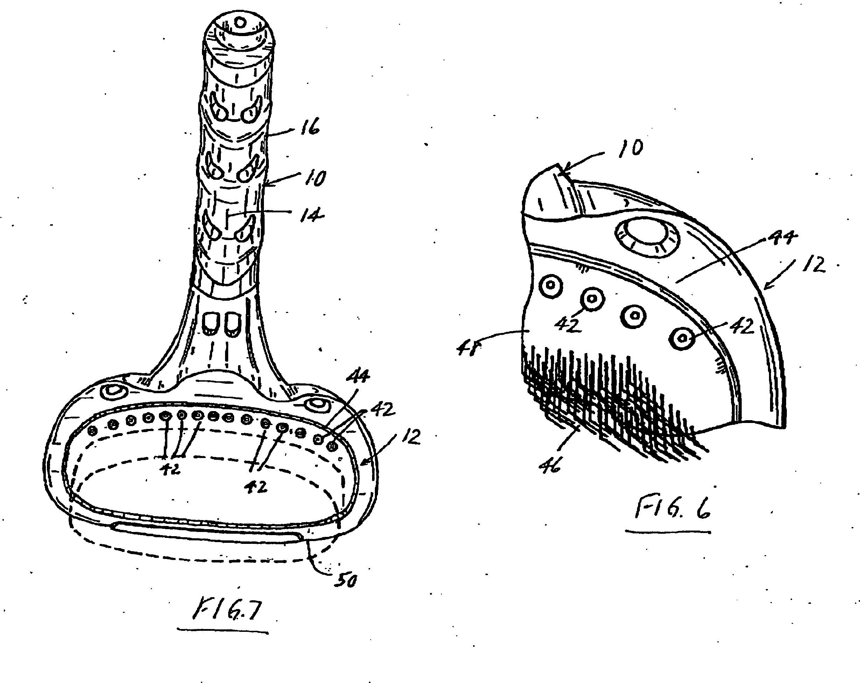

Der Kopf 12 der Bürstenanordnung gemäß den Figuren 6 und 7 trägt eine Reihe von kräftigen Borsten 42 (gemeinsam ein Rakel) an der Unterseite des Kopfes quer zum Rand 44 zum tiefen Rakein von starken Tierhaaren. Zusätzlich trägt der Kopf ein dichtes Muster von feinen Drahtzinken 46 für oberflächliches und rnitteltiefes Kämmen von Tierhaaren. Die starken Borsten 42 und die feinen Drahtzinken 46 sind auf einem Ballen 48 angeordnet, der am Kopf befestigt ist. Der Ballen mit den Zinken 42 und 46 kann vom Kopf abnehmbar ausgebildet sein. Die feinen Drahtzimken 46 halten gelöstes Tierhaar fest, so dass dieses nicht in den Bereich zerstreut wird, in dem das

Tier gepflegt wird. Der Ballen kann in unterschiedlichen Formen ausgebildet sein, die eine oder beide Arten der Zinken halten können. Die Pflegefläche (untere Fläche) 50 kann um eine Achse rechfinrinklig zur Längsachse des Handgriffs gebogen sein, um eine konvexe Arbeitsfläche gemäß Fig. 1 zu bilden, um das Ziehen der Bürste über das Tier zu erleichtern. Der Kopf 12 ist in einem Winkel in Bezug auf den Handgriff ausgebildet, um den Komfort und die Bequemlichkeit der Verwendung der Bürstenanordnung zu verbessern. Die Kopfachse kann außerdem parallel zur Ebene der Oberseite des Handgriffs ausgebildet sein.

Die vorliegende Erfindung ergibt für den Benutzer eine Reihe von Vorteilen. Ein Aspekt dieser Erfindung ist, dass die Bürste leicht ist. Ein anderer besteht darin, dass die Bürste leicht zu bedienen und zu manipulieren ist. Femer bedeutet die Bürste keine Belastung der Handmuskeln oder der Handgelenkbänder, die sich anderenfalls bei wiederholter Aktion ergeben, die beim Pflegen eines Tieres auftreten. Noch ein anderer Aspekt der Erfindung liegt darin, dass der separate Kamm bequem abgelegt werden kann, was insbesondere bei speziellen Pflegebetätigungen hilfreich ist.

Die Bürstenanordnung kann in einer Reihe von unterschiedlichen Größen ausgebildet sein, um sowohl kleine als auch große Tiere wie z. B. Katzen und Hunde zu pflegen. Wenn sie für kleine Tiere verwendet wird, trägt der Ballen normalerweise nur die feineren Kammzinken 46, während für größere Tiere der Ballen vorzugsweise sowohl starke als auch feine Zinken aufinreist. In einer noch anderen Alternative kann das Polster frei von dünnen Zinken sein, und stattdessen eine gummiartige Oberfläche zum Pflegen des Tierfells enthalten.

Nach der Beschreibung verschiedener Aspekte wenigstens einer Ausführungsform der vorliegenden Erfindung ist darauf hinzuweisen, dass verschiedene Änderungen, Modifikationen und Verbesserungen dem Fachmann offen stehen. Solche Änderungen, Modifikationen und Verbesserungen sind Teil der Offenbarung und liegen im Schutzbereich der Erfindung. Die vorstehende Beschreibung und die Zeichnungen sind daher nur als Ausführungsbeispiel zu betrachten und beschränken die Erfindung gemäß den nachstehenden Ansprüchen und ihren Äquivalenten in keiner Weise.

### Bezugszeichen

- 10: Handgriff
- 12: Bürstenkopf
- 14: Fläche
- 15: Fingerkontur
- 16: Kontur
- 17: Kunststoff
- 18: Daumenvertiefung
- 20: Spalt
- 22: Fläche
- 24: Kamm
- 26: Kante
- 28: Ausnehmung
- 29: Handgriff
- 36: Zinken
- 38: Wölbung
- 42: Borsten
- 44: Rand
- 46: Drahtzinken / Kammzimken
- 48: Ballen

## Patentansprüche

1. Tierpflegebürste mit einem Handgriff (10), einem Spalt (20) in dem Handgriff und einem Pflegekamm (24), der in dem Spalt aufnehmbar ist, sowie einem an dem Handgriff befestigten Bürstenkopf (12), **dadurch gekennzeichnet, dass** der Bürstenkopf (12) eine konvexe Arbeitsfläche aufweist, die eine Reihe von dünnen und flexiblen Kammzinken (46) enthält und eine Reihe von im Wesentlichen parallel angeordneter Borsten (42) quer zur Längsrichtung des Handgriffs angeordnet ist, die sich nahe dem Ende des Handgriffs am Bürstenkopf befindet.

2. Tierpflegebürste nach Anspruch 1, bei der der Bürstenkopf (12) einen Ballen (48) enthält, der von dem Bürstenkopf (12) abnehmbar ist, wobei die Kammzinken (46) durch den Ballen gehalten werden.

3. Tierpflegebürste nach Anspruch 1, bei der der Handgriff eine elastomere Oberfläche aufweist.

4. Tierpflegebürste nach Anspruch 3, bei der der Handgriff für den Eingriff der Finger konturiert ist.

5. Tierpflegebürste nach Anspruch 1, bei der die Kammzinken (46) unterschiedliche Größen aufweisen..

6. Tierpflegebürste nach Anspruch 1, bei der die Kammzinken (46) als feine Drahtzinken für Oberflächen- und mitteltiefes Haar ausgebildet sind.

## Claims

1. An animal grooming brush with a handle (10), a slot (20) in the handle and a grooming comb (24), housed in the slot, and a brush head (12) attached to the handle, **characterised in that** the brush head (12) has a convex working area comprising several thin and flexible combing tines (46) as well as several generally parallel tines (42) arrayed transverse to the longitudinal direction of the handle and adjacent the end of the handle attached to the head.

2. An animal grooming brush according to claim 1 wherein the brush head (12) contains a pad (48), which is detachably connected to the brush head (12), whereas the combing tines (46) are carried by the pad.

3. An animal grooming brush according to claim 1 wherein the handle has a soft elastomeric surface.

4. An animal grooming brush according to claim 3 wherein the handle is contoured for engaging the fingers.

5. An animal grooming brush according to claim 1 wherein the combing tines (46) are of different sizes.

6. An animal grooming brush according to claim 1 wherein the combing tines (46) are fine wire tines for surface and middle depth hair.

## Revendications

1. Brosse pour animaux comprenant une poignée (10), une fente (20) dans la poignée et un peigne (24) qui est apte à être logé dans la fente, ainsi qu'une tête de brosse (12) fixée à la poignée, **caractérisée en ce que** la tête de brosse (12) présente une surface de travail convexe qui contient une série de dents (46) fines et flexibles, et il est prévu une série de crins (42) sensiblement parallèles, qui est disposée transversalement par rapport au sens longitudinal de la poignée et qui se trouve près de l'extrémité de celle-ci, sur la tête de la brosse.

2. Brosse pour animaux selon la revendication 1, dans laquelle la tête de brosse (12) contient une protubérance (48) qui peut être enlevée de ladite tête (12), les dents (46) du peigne étant tenues par ladite protubérance.

3. Brosse pour animaux selon la revendication 1, dans laquelle la poignée présente une surface en élastomère.

4. Brosse pour animaux selon la revendication 3, dans laquelle la poignée a des contours qui permettent une bonne prise à l'aide des doigts.

5. Brosse pour animaux selon la revendication 1, dans laquelle les dents (46) présentent différentes tailles.

6. Brosse pour animaux selon la revendication 1, dans laquelle les dents (46) du peigne sont conçues comme des dents métalliques fines pour les poils situés en surface et à une profondeur moyenne.
